Europäisches Patentamt

European Patent Office

Office européen des brevets

(11) Publication number: **0 311 067 B1**

# EUROPEAN PATENT SPECIFICATION

(45) Date of publication of patent specification: **26.08.92**    (51) Int. Cl.⁵: **A61K 9/24**, A61K 31/445, A61K 31/135

(21) Application number: **88116540.1**

(22) Date of filing: **06.10.88**

(54) **Pharmaceutical composition for piperidinoalkanol-decongestant combination.**

(30) Priority: **07.10.87 US 105939**
        **24.06.88 US 211308**

(43) Date of publication of application:
**12.04.89 Bulletin 89/15**

(45) Publication of the grant of the patent:
**26.08.92 Bulletin 92/35**

(84) Designated Contracting States:
**AT BE CH DE ES FR GB GR IT LI LU NL SE**

(56) References cited:
**EP-A- 0 111 144**
**US-A- 3 558 768**

(73) Proprietor: **MERRELL DOW PHARMACEUT-
ICALS INC.
2110 East Galbraith Road
Cincinnati Ohio 45215-6300(US)**

(72) Inventor: **Webb, Norval E.
5373 Cincinnati-Dayton Road
Middletown Ohio 45044(US)**
Inventor: **Hammer, Gregory V.
7234 Adena Court
West Chester Ohio 45247(US)**

(74) Representative: **Vossius & Partner
Siebertstrasse 4 P.O. Box 86 07 67
W-8000 München 86(DE)**

(74) Representative: **Sgarbi, Renato et al
GRUPPO LEPETIT S.p.A. Patent and Trade-
mark Department Via Roberto Lepetit, 34
I-21040 Gerenzano (Varese)(IT)**

## Description

Various piperidinoalkanol derivatives are disclosed in U.S. Patents 3,878,217, 4,254,129 and 4,285,957 as compounds useful as antihistamines, antiallergy agents, and bronchodilators. Included within the scope of these generically defined piperidinoalkanols is $\alpha$-[4-(1,1-dimethylethyl)-phenyl]-4-(hydroxydiphenylmethyl) -1-piperidinebutanol which is commercially available as a pharmaceutical composition in solid unit dosage form for the treatment of patients with symptoms of seasonal allergic rhinitis. These antihistamines are generally effective when administered orally in unit dosage form on a twice a day dosage schedule wherein the unit dosage form provides immediate-release of the active medicament. For example, the recommended dosage for $\alpha$-[4-(1,1-dimethylethyl)phenyl]-4-(hydroxydiphenylmethyl)-1-piperidinebutanol in humans is 60 mg. b.i.d.

Various sympathomimetic drugs such as pseudoephedrine, phenylephrine, and phenylpropanolamine, are recognized by those skilled in the art as therapeutic agents effective in the relief of nasal congestion and are commonly administered concomitantly with antihistamines for relief of nasal congestion associated with allergic rhinitis. These sympathomimetic drugs are generally effective when administered orally in unit dosage form on a four times a day dosage schedule wherein the unit dosage form provides immediate-release of the active medicament. For example, the recommended dosage for pseudoephedrine hydrochloride in adults is 60 mg every 6 hr (q.i.d.). In addition, unit dosage forms containing sympathomimetic drugs can be formulated to provide prolonged release of the active medicament so as to allow the effective daily dose to be administered on a less frequent dosage schedule. For example, the recommended dosage for pseudoephedrine hydrochloride in a prolonged-release formulation can be 120 mg. b.i.d.

Accordingly, the present invention relates to a pharmaceutical composition in the form of a multiple-compression tablet comprising a discrete zone made from a formulation which provides sustained-release of a therapeutically effective decongestant amount of a sympathomimetic drug and a discrete zone made from a different formulation which provides immediate release of a therapeutically effective antihistaminic amount of a piperidinoalkanol and, optionally, a therapeutically effective decongestant amount of a sympathomimetic drug.

More specifically, the present invention provides a pharmaceutical composition in the form of a multiple-compression tablet comprising

(a) a discrete zone made from Formulation (A) which comprises a carrier base material combined with 1 to 200 mg a sympathomimetic drug, or a pharmaceutically acceptable salt thereof, the carrier base material being a mixture of (i) one or more pharmaceutically acceptable water-soluble nonionic cellulose ethers in an amount from 18% to 50% by weight of Formulation (A), (ii) one or more pharmaceutically acceptable anionic surfactants in an amount from 2% to 20% by weight of Formulation (A), and (iii) one or more other pharmaceutically acceptable excipients, and

(b) a discrete zone made from Formulation (B) which comprises a second carrier base material combined with 0.1-140 mg of a piperidinoalkanol, or a pharmaceutically acceptable salt thereof, the second carrier base being a mixture of (i) calcium carbonate in an amount from 0.5% to 25% by weight of Formulation (B), (ii) one or more pharmaceutically acceptable nonionic surfactants in an amount from 1% to 10% by weight of Formulation (B), and (iii) one or more other pharmaceutically acceptable excipients, wherein Formulation (B) optionally also contains 1 to 200 mg of a sympathomimetic drug, or a pharmaceutically acceptable salt thereof;

with the proviso that when said pharmaceutical composition is in the form of a compression-coated tablet, the inner core zone is made with Formulation (A) and the outer coat zone is made with Formulation (B).

As used herein, the term "sympathomimetic drug" refers to those sympathomimetic agents which are therapeutically effective in providing relief of nasal congestion in a patient suffering therefrom including, but not limited to, pseudoephedrine, phenylephrine, and phenylpropanolamine. As is well recognized and appreciated by those skilled in the art, these sympathomimetic drugs can be used according to the present invention as free amines or as pharmaceutically acceptable salts thereof. The term " pharmaceutically acceptable salts " encompasses both organic and inorganic acid addition salts including, for example, those prepared from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. The preferred acid addition salts are those prepared from hydrochloric acid, sulfuric acid or tartaric acid. Any of the above salts are prepared by conventional methods well known in the art.

A therapeutically effective decongestant amount of a sympathomimetic drug is that amount which produces the desired decongestant therapeutic response upon oral administration and can be readily determined by one skilled in the art by the use of conventional techniques and by observing results obtained under analogous circumstances. In determining the therapeutically effective amount, a number of factors are considered, including but not limited to: the particular compound administered; the bioavailability characteristics of the pharmaceutical composition administered; the dose regimen selected; and other relevant circumstances.

A therapeutically effective decongestant amount of a sympathomimetic drug will vary from 1 mg to 200 mg. Preferred amounts will vary from 5 mg to 150 mg.

It is understood that a therapeutically effective decongestant amount of a sympathomimetic drug is present in Formulation (A) and, optionally, in Formulation (B) of the pharmaceutical composition of the present invention. The carrier base material of Formulation (A) provides a prolonged or sustained release of the active medicament whereas the carrier base material of Formulation (B) provides an immediate release of the active medicament(s). As used herein, the term "sustained-release" refers to a property of the pharmaceutical composition wherein the absorption and bioavailability of the active medicament is maintained in a time-release pattern such that therapeutically effective decongestant amounts of the sympathomimetic drug are bioavailable over an extended period of time. The term "immediate-release" refers to a property of the pharmaceutical composition wherein the entire dose of active medicament is made bioavailable without substantial delay.

Where therapeutically effective decongestant amounts of a sympathomimetic drug are present in both Formulation (A) and Formulation (B) of the unit dosage form of the present invention, it is preferred that from 50% to 95% of the total dose of the sympathomimetic drug is present in Formulation (A). It is most preferred that from 80% to 95% of the total dose is present in Formulation (A).

In a particularly preferred embodiment of the present invention, with respect to the sympathomimetic drug, about 110 mg of pseudoephedrine hydrochloride is in Formulation (A) and about 10 mg of pseudoephedrine hydrochloride is in Formulation (B).

It is, of course, understood by the present invention that the sympathomimetic drug in Formulation (A) can be different from that in Formulation (B). However, it is preferred that the sympathomimetic drug in Formulation (A) and in Formulation (B) are the same.

Piperidinoalkanol derivatives which are useful as antihistamines are disclosed in U.S. Patents 3,878,217, 4,254,129 and 4,285,957 and these patents are incorporated herein by reference. For purposes of the present invention the preferred piperidinoalkanol is α-[4-(1,1-dimethylethyl)phenyl]-4-(hydroxydiphenylmethyl) -1-piperidinebutanol. These piperidinoalkanols can be used according to the present invention as the free compound or as a pharmaceutically acceptable salt thereof as described in the above patents.

A therapeutically effective antihistaminic amount of a piperidinoalkanol is that amount which produces the desired antihistaminic therapeutic response upon oral administration and can readily be determined by one skilled in the art as described above for the sympathomimetic drugs. The amount will vary from 0.1 mg to 140 mg. The preferred therapeutically effective antihistaminic amount will vary from about 20 mg to about 70 mg with about 60 mg being most preferred.

It is understood that a therapeutically effective antihistaminic amount of a piperidinoalkanol is present in Formulation (B) of the pharmaceutical composition of the present invention. This Formulation (B) provides for immediate release of the active medicament.

In a particularly preferred embodiment of the present invention with respect to the piperidinoalkanol, about 60 mg of α-(4-(1,1-dimethylethyl)-phenyl]-4-(hydroxydiphenylmethyl) -1-piperidinebutanol is present in Formulation (B).

The pharmaceutically acceptable water soluble nonionic cellulose ethers which are effective in the present invention include, but are not limited to, commercially available, high viscosity grades of methylcellulose, including 1,500 and 4,000 mPa•s (cps) viscosity grades of Methocel® A and Metalose® SM, as well as high viscosity grades of hydroxypropylmethylcellulose, including the 4,000 mPa•s (cps) viscosity grades of Methocel® E and Metalose® 60SH, the 4,000 mPa•s (cps) viscosity grades of Methocel® F and Metalose® 65SH, the 5,000, 12,000, 20,000, and 75,000 mPa•s (cps) viscosity grades of Methocel® J and the 4,000, 15,000 and 100,000 mPa•s (cps) viscosity grades of Methocel® K and the 4,000, 15,000 and 39,000 mPa•s (cps) viscosity grades of Metalose® 90SH. Methocel® K4M (2208) is preferred.

The amount of nonionic cellulose ether in Formulation (A) of the pharmaceutical composition of the present invention can vary from 18% to 50% by weight of Formulation (A). Compositions wherein the nonionic cellulose ether makes up from 24% to 36% are preferred.

The pharmaceutically acceptable anionic surfactants which are effective in the present invention include alkali metal sulfates of linear and branched alcohols, ethoxylated alcohols, ethoxylated alkylphenols, ethoxylated acids, ethoxylated amides, oils, fatty esters, etc., alkali metal salts of sulfonates of naphthalene, alkylnaphthalenes, naphthalene condensates, alkyl-substituted benzenes, diphenyl derivatives, $\alpha$-olefins, petroleum, oils, fatty acids, etc., as well as the alkali metal salts of dialkyl sulfosuccinates.

Representative anionic surfactants include sodium or potassium dodecyl sulfate, sodium octadecyl sulfate, sodium sulfated castor oil, sodium dodecylbenzene sulfonate, sodium linear alkylate sulfonate, sodium sulfonated mineral oil, sodium petroleum sulfonate, sodium salt of naphthalenesulfonic acid-formaldehyde condensate, dioctyl sodium sulfosuccinate the the like. Sodium dodecyl sulfate is preferred.

The amount of anionic surfactant in Formulation (A) of the pharmaceutical composition of the present invention can vary from 2% to 20% by weight of the Formulation (A). Compositions wherein the anionic surfactant makes up from 5% to 15% are preferred.

The pharmaceutical acceptable nonionic surfactants which are effective in the present invention includes pharmaceutically acceptable nonionic surfactants known in the art of pharmaceutical science, such as, for example, Polysorbate 80 (also known as Tween 80), and various poloxamers or pluronics, or mixtures thereof. The preferred nonionic surfactant in the pharmaceutical composition of the present invention is Polysorbate 80.

The amount of the nonionic surfactant in Formulation (B) of the pharmaceutical composition of the present invention can vary from 1% to 10% by weight of Formulation (B). Compositions wherein the nonionic surfactant makes up from 2% to 6% are preferred.

The amount of calcium carbonate present in Formulation (B) of the pharmaceutical composition of the present invention can vary from 0.5% to 25% by weight of the Formulation (B). Compositions wherein the calcium carbonate makes up from 5% to 20% are preferred, with compositions wherein the calcium carbonate makes up 15% being most preferred.

Formulation (A) and Formulation (B) of the pharmaceutical compositions of the present invention also contain one or more other pharmaceutically acceptable excipients. These excipients are therapeutically inert ingredients such as are well known and appreciated in the art. Such excipients include, for example, conventional carriers (such as lactose, corn starch or derivatives thereof, talc, stearic acid or salts thereof, methyl cellulose or derivatives thereof including hydroxypropylmethylcellulose, and the like), preserving agents, stabilizing agents, disintegrants, sweetening agents, coloring agents, flavoring agents, antioxidants, buffers, and the like. Selection of a particular ingredient or ingredients and the amounts used can be readily determined by one skilled in the art by reference to standard procedures and practices.

Preferred compositions of the present invention are those wherein pregelatinized corn starch (Starch 1500), microcrystalline cellulose (Avicel®), starch glycolate sodium (Explotab®), and magnesium stearate are present as other pharmaceutically acceptable excipients in Formulation (B). Preferred amounts of the above excipients are from 25% to 45%, 20% to 40%, 1% to 10%, and 0.1% to 1%, respectively, by weight of Formulation (B) with 35%, 30%, 5%, and 0.55%, respectively, being most preferred.

Preferred compositions of the present invention are those wherein colloidal silicon dioxide and zinc stearate are present as other pharmaceutically acceptable excipients in Formulation (A). Preferred amounts of the above excipients are from 0.1% to 5% and from 0.5% to 10%, respectively, by weight of Formulation (A) with 0.5% and 1.5%, respectively, being most preferred.

The ingredients of the pharmaceutical composition according to the present invention are brought together into a form of a multiple-compression tablet for oral administration according to standard practice and procedures well known in the art of pharmaceutical science using conventional formulation and manufacturing techniques.

The multiple-compression tablet of the present invention is a compression tablet which is formed by two or more compression cycles. Each compression cycle uses, alternatively, granulations made from either Formulation (A) or Formulation (B). This results in a multiple-compression tablet which has at least two discrete zones defined by the presence of either Formulation (A) or Formulation (B) in the zone. The multiple-compression tablets of the present invention have at least two discrete zones, one defined by the presence of Formulation (A) and one defined by the presence of Formulation (B). A multiple-compression tablet of the present invention can exist as a layered tablet, as a compression-coated tablet, or as an inlay tablet.

A layered tablet is a tablet which is made up of two or more distinct layers or discrete zones of granulation compressed together with the individual layers lying one on top of another. Layered tablets have the appearance of a sandwich because the edges of each layer or zone is exposed. Such conventional layered tablets are generally prepared by compressing a granulation onto a previously compressed granulation. The operation may be repeated to produce multilayered tablets of more than two layers. A layered tablet of the present invention has at least two layers or discrete zones one of which is made from Formulation (A) and another of which is made from Formulation (B).

A compression-coated tablet is a tablet which is made up of an inner core and and one or more outer coats wherein the inner core is completely surrounded by the outer coat or coats. These tablets have at least two discrete zones of granulation compressed together, i.e., an inner core zone and an outer coat zone. Such tablets, also referred to as presscoat or dry-coated tablets, are prepared by feeding a previously compressed inner core into a special tableting machine and compressing one or more other granulation coats around the preformed inner core. A compression-coated tablet of the present invention has an inner core or inner core zone made from Formulation (A) and at least one press-coat or press-coat zone made from Formulation (B}.

A variation of the compression-coated tablet is the inlay tablet, also referred to as a dot, or bull's-eye tablet. Instead of an inner core zone being completely surrounded by an outer coat, one surface of the zone corresponding to an inner core zone is exposed. These tablets have at least two discrete zones of granulation compressed together, i.e., an inlay zone and a base zone. The preparation of inlay tablets is similar to the preparation of compression-coated tablets except that a surface of coating is eliminated. An inlay tablet of the present invention has at least two discrete zones, one of which is made from Formulation (A) and another of which is made from Formulation (B).

In a preferred embodiment of the present invention, the multiple-compression tablet is in the form of a compression-coated tablet. Compression-coated tablets of the present invention can generally be manufactured by a three-step procedure involving preparation of the inner core and outer coat granulations from Formulation (A) and Formulation (B), respectively, compressing the inner core granulation to provide the inner core tablet, and subsequently compressing an outer coat granulation around the inner core tablet so as to provide two discrete zones with the inner core zone defined by Formulation (A) and the outer coat zone defined by Formulation (B).

It is of course understood that the multiple-compression tablets produced according to the present invention can be film coated using standard ingredients and procedures commonly used and well known in the art of pharmaceutical science. It is contemplated that tablets so coated are within the scope of the present invention.

In a preferred embodiment of the present invention tablets are formulated and manufactured using the procedure as described in Example 1. This example is not intended to limit the scope of the invention in any way.

EXAMPLE 1

Preparation of Tablets Containing $\alpha$-[4-(1,1-Dimethylethyl)phenyl]-4-(hydroxydiphenylmethyl)-1-piperidinebutanol and Pseudoephedrine Hydrochloride

Step A: Formulation (A)

Combine 110 g pseudoephedrine hydrochloride (milled if necessary), 108 g of hydroxypropylmethylcellulose (2208), 15 g of sodium dodecyl sulfate (sodium lauryl sulfate), 1.5 g of silicon dioxide and mix thoroughly using a high-intensity type mixture such as the Collette Gral. Pass 1.5 g of zinc stearate through a 30-mesh screen, add to the above mixture and continue mixing.

Form compacted tablets (slugs) using a tablet press. Mill the slugs and separate the granulation into coarse and fine granules by using a screening device. The fine powder mix may be compacted first to facilitate slugging. Reslug, mill and screen the coarse granules as necessary, to obtain a suitable granulation.

Pass 3.0 g of zinc stearate through a 30-mesh screen, add to the fine granules above, and blend in a mixer such as a V-blender.

Step B: Formulation (B)

Combine 60 g of $\alpha$-[4-(1,1-dimethylethyl)-phenyl]-4-(hydroxydiphenylmethyl -1-piperidinebutanol 85.7 g of microcrystalline cellulose, 125 g of pregelatinized corn starch, and 87.5 g of calcium carbonate and mix thoroughly. Prepare a granulating solution by dissolving 17.5 g of Polysorbate 80 in about 177.3 g of purified water and add to the above mixture. Mix until well formed granules are produced. Wet screen the granulation if necessary.

Dry the granulation to a moisture level of not more than 3% (O'Haus moisture measuring device). Screen the dried granulation into coarse and fine granules and mill the coarse granules using size reduction equipment such as a Fitzmill. Combine the milled coarse granules and the fine granules and mix in a mixer. To this granulation, add 191.3 g of a mixture of 10 g of pseudoephedrine hydrochloride, 87.5 g of microcrystalline cellulose, 62.5 g of pregelatinized corn starch and 31.3 g of starch glycolate sodium and mix thoroughly. Add 3.0 g of magnesium stearate (screened) and continue mixing until a suitable mixture is obtained.

Step C: Tablet Compression

a. Press-coat tablet: Feed the inner core granulation [Formulation (A)] into a tablet press and form inner core tablet with an average weight of 290-310 mg, a hardness of 1-7 kp (Schleuninger), and a thickness of 4.5-5.5 mm.

Feed the inner core tablet into a tablet press charged with the outer coat granulation [Formulation (B)] and compress the outer coat around the inner core tablet to provide the finished compression-coated tablet. About 1000 tablets are made with an average weight of 835-905 mg, a hardness of 5-15 kp (Schleuninger) and friability of NMT 1%.

b. Layered tablet: Feed the granulations prepared from Formulation (A) and Formulation (B) into a tablet press suitable for preparing conventional multilayer tablets. Prepare a layered tablet with one layer made from Formulation (A) and another layer made from Formulation (B). The layer made from Formulation (A) is made with an average weight of 290-310 mg. About 1000 tablets are made with an average weight of 835-905 mg.

## Claims

**Claims for the following Contracting States : AT, BE, CH, DE, FR, GB, IT, LI, LU, NL, SE**

1. A pharmaceutical composition in the form of a multiple-compression tablet comprising
   (a) a discrete zone made with Formulation (A) which comprises a carrier base material combined with 1 to 200 mg of a sympathomimetic drug, or a pharmaceutically acceptable salt thereof, the carrier base material being a mixture of (i) one or more pharmaceutically acceptable water-soluble nonionic cellulose ethers in an amount from 18% to 50% by weight of Formulation (A), (ii) one or more pharmaceutically acceptable anionic surfactants in an amount from 2% to 20% by weight of Formulation (A), and (iii) one or more other pharmaceutically acceptable excipients, and
   (b) a discrete zone made with Formulation (B) which comprises a second carrier base material combined with 0.1 to 140 mg of a piperidinoalkanol, or a pharmaceutically acceptable salt thereof, the second carrier base being a mixture of (i) calcium carbonate in an amount from 0.5% to 25% by weight of Formulation (B), (ii) one or more pharmaceutically acceptable nonionic surfactants in an amount from 1% to 10% by weight of Formulation (B), and (iii) one or more other pharmaceutically acceptable excipients, wherein Formulation (B) optionally also contains 1 to 200 mg of a sympathomimetic drug, or a pharmaceutically acceptable salt thereof;
   with the proviso that when said pharmaceutical composition is in the form of a compression-coated tablet, the inner core zone is made with Formulation (A) and the outer coat zone is made with Formulation (B).

2. A composition of Claim 1 in the form of a compression-coated tablet.

3. A composition of Claim 2 wherein the sympathomimetic drug is pseudoephedrine.

4. A composition of Claim 2 wherein the piperidinoalkanol is $\alpha$-[4-(1,1-dimethylethyl)-phenyl]-4-(hydroxydiphenylmethyl) -1-piperidinebutanol.

5. A composition of Claim 2 wherein the sympathomimetic drug is pseudoephedrine and the piperidinoalkanol is $\alpha$-(4-(1,1-dimethylethyl)-phenyl]-4-(hydroxydiphenylmethyl) -1-piperidinebutanol.

6. A composition of Claim 5 wherein the inner core zone contains about 110 mg of pseudoephedrine hydrochloride and outer coat zone contains about 60 mg of $\alpha$-(4-(1,1-dimethylethyl) phenyl]-4-(hydroxydiphenylmethyl) -1-piperidinebutanol and about 10 mg of pseudoephedrine hydrochloride.

7. A composition of Claim 6 wherein the inner core zone carrier base material contains from 5% to 15% sodium dodecyl sulfate as the anionic surfactant.

8. A composition of Claim 6 wherein the outer coat zone carrier base material contains from 2% to 6% of Polysorbate 80 as the nonionic surfactant and from 5% to 20% of calcium carbonate.

9. A composition of Claim 8 wherein the outer coat zone carrier base material contains about 15% of calcium carbonate.

10. A composition of Claim 7 wherein the outer coat zone carrier base material contains from 2% to 6% of Polysorbate 80 as the nonionic surfactant and from 5% to 20% of calcium carbonate.

11. A composition of Claim 10 wherein the outer coat zone carrier base material contains about 15% of calcium carbonate.

12. A process of making a pharmaceutical composition in the form of a multiple-compression tablet according to any of claims 1 to 11, comprising

(a) preparing Formulation (A) by admixing a carrier base material and a therapeutically effective decongestant amount of a sympathomimetic drug, or a pharmaceutically acceptable salt thereof, the carrier base material being a mixture of (i) one or more pharmaceutically acceptable water-soluble nonionic cellulose ethers in an amount from about 18% to about 50% by weight of Formulation (A), (ii) one or more pharmaceutically acceptable anionic surfactants in an amount from about 2% to about 20% by weight of Formulation (A), and (iii) one or more other pharmaceutically acceptable excipients,

(b) preparing Formulation (B) by admixing a second carrier base material and a therapeutically effective antihistaminic amount of a piperidinoalkanol, or a pharmaceutically acceptable salt thereof, the second carrier base being a mixture of (i) calcium carbonate in an amount from about 0.5 to about 25% by weight of Formulation (B), (ii) one or more pharmaceutically acceptable nonionic surfactants in an amount from about 1% to about 10% by weight of Formulation (B), and (iii) one or more other pharmaceutically acceptable excipients, wherein Formulation (B) optionally also contains a therapeutically effective decongestant amount of a sympathomimetic drug, or a pharmaceutically acceptable salt thereof; and

(c) multiply compressing Formulations (A) and (B) so as to provide a tablet having one or more discrete zones made with Formulation (A) and one or more discrete zones made with Formulation (B), with the proviso that when said pharmaceutical composition is in the form of a compression-coated tablet with an inner core zone and an outer coat zone, the inner core zone is made with Formulation (A) and the outer coat zone is made with Formulation (B).

**Claims for the following Contracting States : ES, GR**

1. A process of making a pharmaceutical composition in the form of a multiple-compression tablet comprising

(a) a discrete zone made with Formulation (A) which comprises a carrier base material combined with 1 to 200 mg of a sympathomimetic drug, or a pharmaceutically acceptable salt thereof, the carrier base material being a mixture of (i) one or more pharmaceutically acceptable water-soluble nonionic cellulose ethers in an amount from 18% to 50% by weight of Formulation (A), (ii) one or more pharmaceutically acceptable anionic surfactants in an amount from 2% to 20% by weight of Formulation (A), and (iii) one or more other pharmaceutically acceptable excipients, and

(b) a discrete zone made with Formulation (B) which comprises a second carrier base material combined with 0.1 to 140 mg of a piperidinoalkanol, or a pharmaceutically acceptable salt thereof, the second carrier base being a mixture of (i) calcium carbonate in an amount from 0.5% to 25% by weight of Formulation (B), (ii) one or more pharmaceutically acceptable nonionic surfactants in an amount from 1% to 10% by weight of Formulation (B), and (iii) one or more other pharmaceutically acceptable excipients, wherein Formulation (B) optionally also contains 1 to 200 mg of a sympathomimetic drug, or a pharmaceutically acceptable salt thereof,

which process comprises

(a) preparing Formulation (A) by admixing a carrier base material and a therapeutically effective decongestant amount of a sympathomimetic drug, or a pharmaceutically acceptable salt thereof, the carrier base material being a mixture of (i) one or more pharmaceutically acceptable water-soluble nonionic cellulose ethers in an amount from about 18% to about 50% by weight of Formulation (A), (ii) one or more pharmaceutically acceptable anionic surfactants in an amount from about 2% to about 20% by weight of Formulation (A), and (iii) one or more other pharmaceutically acceptable excipients,

(b) preparing Formulation (B) by admixing a second carrier base material and a therapeutically effective antihistaminic amount of a piperidinoalkanol, or a pharmaceutically acceptable salt thereof, the second carrier base being a mixture of (i) calcium carbonate in an amount from about 0.5 to about 25% by weight of Formulation (B), (ii) one or more pharmaceutically acceptable nonionic surfactants in an amount from about 1% to about 10% by weight of Formulation (B), and (iii) one or more other pharmaceutically acceptable excipients, wherein Formulation (B) optionally also contains a therapeutically effective decongestant amount of a sympathomimetic drug, or a pharmaceutically acceptable salt thereof; and

(c) multiply compressing Formulations (A) and (B) so as to provide a tablet having one or more discrete zones made with Formulation (A) and one or more discrete zones made with Formulation (B), with the proviso that when said pharmaceutical composition is in the form of a compression-coated tablet with an inner core zone and an outer coat zone, the inner core zone is made with Formulation (A) and the outer coat zone is made with Formulation (B).

2. A process according to claim 1 wherein the composition is in the form of a compression-coated tablet.

3. A process according to Claim 2 wherein the sympathomimetic drug is pseudoephedrine.

4. A process according to Claim 2 wherein the piperidinoalkanol is $\alpha$-[4-(1,1-dimethylethyl)-phenyl]-4-(hydroxydiphenylmethyl)-1-piperidinebutanol.

5. A process according to Claim 2 wherein the sympathomimetic drug is pseudoephedrine and the piperidinoalkanol is $\alpha$-[4-(1,1-dimethylethyl) phenyl]-4-(hydroxydiphenylmethyl) -1-piperidinebutanol.

6. A process according to claim 5 wherein in the composition the inner core zone contains about 110 mg of pseudoephedrine hydrochloride and outer coat zone contains about 60 mg of $\alpha$-[4-(1,1-dimethylethyl) phenyl]-4-(hydroxydiphenylmethyl) -1-piperidinebutanol and about 10 mg of pseudoephedrine hydrochloride.

7. A process according to claim 6 wherein in the composition the inner core zone carrier base material contains from 5% to 15% sodium dodecyl sulfate as the anionic surfactant.

8. A process according to claim 6 wherein in the composition the outer coat zone carrier base material contains from 2% to 6% of Polysorbate 80 as the nonionic surfactant and from 5% to 20% of calcium carbonate.

9. A process according to Claim 8 wherein in the composition the outer coat zone carrier base material contains about 15% of calcium carbonate.

10. A process according to Claim 7 wherein in the composition the outer coat zone carrier base material contains from 2% to 6% of Polysorbate 80 as the nonionic surfactant and from 5% to 20% off calcium carbonate.

11. A process according to Claim 10 wherein in the composition the outer coat zone carrier base material contains about 15% of calcium carbonate.

**Patentansprüche**

**Patentansprüche für folgende Vertragsstaaten : AT, BE, CH, DE, FR, GB, IT, LI, LU, NL, SE**

1. Arzneimittel in Form einer Mehrschicht-Preßtablette, enthaltend

    (a) eine getrennte Zone, die aus Formulierung (A) hergestellt ist, die ein Trägerbasismaterial kombiniert mit 1 bis 200 mg eines sympathomimetischen Arzneistoffes oder eines pharmazeutisch verträglichen Salzes davon enthält, wobei das Trägerbasismaterial ein Gemisch darstellt aus (i) einem oder mehreren pharmazeutisch verträglichen wasserlöslichen nichtionischen Celluloseethern in einer Menge von 18 bis 50 Gew.% der Formulierung (A), (ii) einem oder mehreren pharmazeutisch verträglichen anionischen Tensiden in einer Menge von 2 bis 20 Gew.% der Formulierung (A) und (iii) einem oder mehreren anderen pharmazeutisch verträglichen Vehikeln, und

    (b) eine getrennte Zone, die aus Formulierung (B) hergestellt ist, die ein zweites Trägerbasismaterial kombiniert mit 0,1 bis 140 mg eines Piperidinoalkanols oder eines pharmazeutisch verträglichen Salzes davon enthält, wobei die zweite Trägerbasis ein Gemisch darstellt aus (i) Calciumcarbonat in einer Menge von 0,5 bis 25 Gew.% der Formulierung (B), (ii) einem oder mehreren pharmazeutisch verträglichen nichtionischen Tensiden in einer Menge von 1 bis 10 Gew.% der Formulierung (B) und (iii) einem oder mehreren anderen pharmazeutisch verträglichen Vehikeln, wobei die Formulierung (B) gegebenenfalls auch 1 bis 200 mg eines sympathomimetischen Arzneistoffes oder eines pharmazeutisch verträglichen Salzes davon enthält;

    mit der Maßgabe, daß dann, wenn das Arzneimittel in Form einer preßbeschichteten Tablette vorliegt, die innere Kernzone mit Formulierung (A) und die Außenschichtzone mit Formulierung (B) hergestellt wird.

2. Mittel nach Anspruch 1 in Form einer preßbeschichteten Tablette.

3. Mittel nach Anspruch 2, wobei der sympathomimetische Arzneistoff Pseudoephedrin ist.

4. Mittel nach Anspruch 2, wobei das Piperidinoalkanol α-[4-(1,1-Dimethylethyl)phenyl]-4-(hydroxydiphenylmethyl)-1-piperidinbutanol ist.

5. Mittel nach Anspruch 2, wobei der sympathomimetische Arzneistoff Pseudoephedrin und das Piperidinoalkanol α-[4-(1,1-Dimethylethyl)-phenyl]-4-(hydroxydiphenylmethyl) -1-piperidinbutanol sind.

6. Mittel nach Anspruch 5, wobei die innere Kern-zone etwa 110 mg Pseudoephedrinhydrochlo-rid enthält und die Außenschichtzone etwa 60 mg α-[4-(1,1-Dimethylethyl)phenyl]-4-(hydroxydiphenylmethyl) -1-piperidinbutanol und etwa 10 mg Pseudoephedrinhydrochlorid enthält.

7. Mittel nach Anspruch 6, wobei das Trägerbasismaterial der inneren Kernzone 5% bis 15% Natriumdodecylsulfat als anionisches Tensid enthält.

8. Mittel nach Anspruch 6, wobei das Trägerbasismaterial der Außenschichtzone 2% bis 6% Polysorbat 80 als nichtionisches Tensid und 5% bis 20% Calciumcarbonat enthält.

9. Mittel nach Anspruch 8, wobei das Trägerbasismaterial der Außenschichtzone etwa 15% Calciumcarbonat enthält.

10. Mittel nach Anspruch 7, wobei das Trägerbasismaterial der Außenschichtzone 2% bis 6% Polysorbat 80 als nichtionisches Tensid und 5% bis 20% Calciumcarbonat enthält.

11. Mittel nach Anspruch 10, wobei das Trägerbasismaterial der Außenschichtzone etwa 15% Calciumcarbonat enthält.

12. Verfahren zur Herstellung eines Arzneimittels in Form einer Mehrschicht-Preßtablette nach einem der Ansprüche 1 bis 11, umfassend
(a) Herstellung der Formulierung (A) durch Mischen eines Trägerbasismaterials und ei-ner therapeutisch wirksamen kongestions-vermindernden Menge eines sympathomi-metischen Arzneistoffes oder eines pharma-zeutisch verträglichen Salzes davon, wobei das Trägerbasismaterial ein Gemisch darstellt aus (i) einem oder mehreren pharmazeutisch verträglichen wasserlöslichen nichtionischen Celluloseäthern in einer Menge von etwa 18 bis etwa 50 Gew.% der Formulierung (A), (ii) einem oder mehreren pharmazeutisch verträglichen anionisohen Tensiden in einer Menge von etwa 2 bis etwa 20 Gew.% der Formulierung (A) und (iii) einem oder mehreren anderen pharmazeutisch verträglichen Vehikeln.
(b) Herstellung der Formulierung (B) durch Mischen eines zweiten Trägerbasismaterials und einer therapeutisch wirksamen antihi-staminischen Menge eines Piperidinoalkan-ols oder eines pharmazeutisch verträglichen Salzes davon, wobei die zweite Trägerbasis ein Gemich darstellt aus (i) Calciumcarbonat in einer Menge von etwa 0,5 bis etwa 25 Gew.% der Formulierung (B), (ii) einem oder mehreren pharmazeutisch verträglichen nichtionischen Tensiden in einer Menge von etwa 1 bis etwa 10 Gew.% der Formulierung (B) und (iii) einem oder mehreren anderen pharmazeutisch verträglichen Vehikeln, wobei die Formulierung (B) gege-benenfalls auch eine therapeutisch wirksa-me kongestionsvermindernde Menge eines sympathomimetischen Arzneisstoffs oder eines pharmazeutisch verträglichen Salzes davon enthält; und
(c) Mehrfachverpressung der Formulierungen (A) und (B) derart, daß eine Tablette erhalten wird, mit einer oder mehreren getrennten Zonen, hergestellt aus Formulierung(A), und einer oder mehreren getrennten Zonen, hergestellt aus Formulierung (B) mit der Maßgabe, daß dann, wenn das Arzneimittel in Form einer preßbeschichteten Tablette mit einer inneren Kernzone und einer Außenschichtzone vorliegt, die innere Kernzone mit Formulierung (A) und die Außenschichtzone mit Formulierung (B) hergestellt wird.

**Patentansprüche für folgende Vertragsstaaten : ES, GR**

1. Verfahren zur Herstellung eines Arzneimittels in Form einer Mehrschicht-Preßtablette, enthaltend
(a) eine getrennte Zone, die aus Formulierung (A) hergestellt ist, die ein Trägerbasismaterial kombiniert mit 1 bis 200 mg eines svmpathomietischen Arzneistoffes oder ei-nes pharmazeutisch verträglichen Salzes davon enthält, wobei das Trägerbasismaterial ein Gemisch darstellt aus (i) einem oder mehreren pharmazeutisch verträglichen wasserlöslichen nichtionischen Celluloseethern in einer Menge von 18 bis 50 Gew.% der Formulierung (A), (ii) einem oder mehre-ren pharmazeutisch verträglichen anioni-schen Tensiden in einer Menge von 2 bis 20 Gew.% der Formulierung (A) und (iii) einem oder mehreren anderen pharmazeutisch verträglichen Vehikeln, und und (b) eine getrennte Zone, die aus Formulierung (B) hergestellt ist, die ein zweites Trägerbasismaterial kombiniert mit 0,1 bis 140 mg eines Piperidinoalkanols oder eines pharmazeutisch verträglichen Salzes davon enthält, wobei die zweite Trägerbasis ein Gemisch darstellt aus (i) Calciumcarbonat in einer Menge von 0,5 bis 25 Gew.% der Formulierung (B), (ii) einem oder mehreren pharmazeutisch verträglichen nichtionischen Tensiden in einer Menge von 1 bis 10 Gew.% der Formulierung (B) und (iii) einem oder mehreren anderen pharmazeutisch verträglichen Vehikeln wobei die Formulierung (B) gegebenenfalls auch 1 bis 200 mg eines sympathomimetischen Arzneistoffes oder eines pharmazeutisch verträglichen Salzes davon enthält,

wobei das Verfahren umfaßt:

(a) Herstellung der Formulierung (A) durch Mischen eines Trägerbasismaterials und einer therapeutisch wirksamen kongestionsvermindernden Menge eines sympathomimetischen Arzneistoffs oder eines pharmazeutisch verträglichen Salzes davon, wobei das Trägerbasismaterial ein Gemisch darstellt aus (i) einem oder mehreren pharmazeutisch verträglichen wasserlöslichen nichtionischen Celluloseäthern in einer Menge von etwa 18 bis etwa 50 Gew.% der Formulierung (A), (ii) einem oder mehreren pharmazeutisch verträglichen anionischen Tensiden in einer Menge von etwa 2 bis etwa 20 Gew.% der Formulierung (A) und (iii) einem oder mehreren anderen pharmazeutisch verträglichen Vehikeln.

(b) Herstellung der Formulierung (B) durch Mischen eines zweiten Trägerbasismaterials und einer therapeutisch wirksamen antihistaminischen Menge eines Piperidinoalkanols oder eines pharmazeutisch verträglichen Salzes davon, wobei die zweite Trägerbasis ein Gemisch darstellt aus (i) Calciumcarbonat in einer Menge von etwa 0,5 bis etwa 25 Gew.% der Formulierung (B), (ii) einem oder mehreren pharmazeutisch verträglichen nichtionischen Tensiden in einer Menge von etwa 1 bis etwa 10 Gew.% der Formulierung (B) und (iii) einem oder mehreren anderen pharmazeutisch verträglichen Vehikeln, wobei die Formulierung (B) gegebenenfalls auch eine therapeutisch wirksame kongestionsvermindernde Menge eines sympathomimetischen Arzneistoffs oder eines pharmazeutisch verträglichen Salzes davon enthält; und

(c) Mehrfachverpressung der Formulierungen (A) und (B) derart, daß eine Tablette erhalten wird, mit einer oder meheren getrennten Zonen, hergestellt aus Formulierung (A), und einer oder mehreren Zonen, hergestellt aus Formulierung (B), mit der Maßgabe, daß dann, wenn das Arzneimittel in Form einer preßbeschichteten Tablette mit einer inneren Kernzone und einer Außenschichtzone vorliegt, die innere Kernzone mit Formulierung (A) und die Außenschichtzone mit Formulierung (B) hergestellt wird.

2. Verfahren nach Anspruch 1, wobei das Mittel in Form einer preßbeschichteten Tablette vorliegt.

3. Verfahren nach Anspruch 2, wobei der sympathomimetische Arzneistoff Pseudoephedrin ist.

4. Verfahren nach Anspruch 2, wobei das Piperidinoalkanol α-[4-(1,1-Dimethyläthyl)phenyl]-4-(hydroxydiphenylmethyl) -1-piperidinbutanol ist.

5. Verfahren nach Anspruch 2, wobei der sympathomimetische Arzneistoff Pseudoephedrin und das Piperidinoalkanol α-[4-(1,1-Dimethylethyl)-phenyl]-4-(hydroxydiphenylmethyl) -1-piperidinbutanol sind.

6. Verfahren nach Anspruch 5, wobei in dem Mittel die innere Kernzone etwa 110 mg Pseudoephedrinhydrochlorid enthält und die Außenschichtzone etwa 60 mg α-[4-(1,1-Dimethyläthyl)phenyl]-4-(hydroxydiphenylmethyl) -1-piperidinbutanol und etwa 10 mg Pseudoephedrinhydrochlorid enthält.

7. Verfahren nach Anspruch 6, wobei in dem Mittel das Trägerbasismaterial der inneren Kernzone 5% bis 15% Natriumdodecylsulfat als anionisches Tensid enthält.

8. Verfahren nach Anspruch 6, wobei in dem Mittel das Trägerbasismaterial der Außenschichtzone 2% bis 6% Polysorbat 80 als nichtionisches Tensid und 5% bis 20% Calciumcarbonat enthält.

9. Verfahren nach Anspruch 8, wobei in dem Mittel das Trägerbasismaterial der Außenschichtzone etwa 15% Calciumcarbonat enthält.

10. Verfahren nach Anspruch 7, wobei in dem Mittel das Trägerbasismaterial der Außenschichtzone 2% bis 6% Polysorbat 80 als nichtionisches Tensid und 5% bis 20% Calciumcarbonat enthält.

11. Verfahren nach Anspruch 10, wobei in dem Mittel das Trägerbasismaterial der Außenschichtzone etwa 15% Calciumcarbonat enthält.

**Revendications**
**Revendications pour les Etats contractants suivants : AT, BE, CH, DE, FR, GB, IT, LI, LU, NL, SE**

1. Composition pharmaceutique sous la forme d'un comprimé à compression multiple comprenant

   (a) une zone distincte faite d'une Formulation (A) qui comprend une matière de base de support combinée avec 1 à 200 mg d'un médicament sympathomimétique ou d'un de ses sels pharmaceutiquement acceptables, la matière de base de support étant un mélange de (i) un ou plusieurs éthers de cellulose non ioniques solubles dans l'eau pharmaceutiquement acceptables en quantité de 18 à 50 % en poids de la Formulation (A), (ii) un ou plusieurs tensioactifs anioniques pharmaceutiquement acceptables en quantité de 2 à 20 % en poids de la Formulation (A) et (iii) un ou plusieurs autres excipients pharmaceutiquement acceptables et

   (b) une zone distincte faite d'une Formulation (B) qui comprend une seconde matière de base de support combinée avec 0,1 à 140 mg d'un pipéridinoalcanol ou d'un de ses sels pharmaceutiquement acceptables, la seconde base de support étant un mélange de (i) carbonate de calcium en quantité de 0,5 à 25 % en poids de la Formulation (B), (ii) un ou plusieurs tensioactifs non ioniques pharmaceutiquement acceptables en quantité de 1 à 10 % en poids de la Formulation (B) et (iii) un ou plusieurs autres excipients pharmaceutiquement acceptables, la Formulation (B) contenant aussi facultativement 1 à 200 mg d'un médicament sympathomimétique ou d'un de ses sels pharmaceutiquement acceptables ;

   avec la condition que, lorsque ladite composition pharmaceutique est sous la forme d'un comprimé enrobé par compression, la zone de noyau intérieur est faite de la Formulation (A) et la zone d'enrobage extérieur est faite de la Formulation (B).

2. Une composition selon la revendication 1, sous la forme d'un comprimé enrobé par compression.

3. Une composition selon la revendication 2, dans laquelle le médicament sympathomimétique est la pseudoéphédrine.

4. Une composition selon la revendication 2, dans laquelle le pipéridinoalcanol est l'α-[4-(1,1-diméthyléthyl)-phényl]-4-(hydroxydiphénylméthyl) -1-pipéridinebutanol.

5. Une composition selon la revendication 2, dans laquelle le médicament sympathomimétique est la pseudoéphédrine et le pipéridinoalcanol est l'α-[4-(1,1-diméthyléthyl)-phényl]-4-(hydroxydiphénylméthyl) -1-pipéridinebutanol.

6. Une composition selon la revendication 5, dans laquelle la zone de noyau interne contient environ 110 mg de chlorhydrate de pseudoéphédrine et la zone d'enrobage externe contient environ 60 mg d'α-[4-(1,1-diméthyléthyl)-phényl]-4-(hydroxydiphénylméthyl) -1-pipéridinebutanol et environ 10 mg de chlorhydrate de pseudoéphédrine.

7. Une composition selon la revendication 6, dans laquelle la matière de base de support de la zone de noyau interne contient de 5 à 15 % de dodécylsulfate de sodium comme tensioactif anionique.

8. Une composition selon la revendication 6, dans laquelle la matière de base de support de la zone d'enrobage externe contient de 2 à 6 % de Polysorbate 80® comme tensioactif non ionique et de 5 à 20 % de carbonate de calcium.

9. Une composition selon la revendication 8, dans laquelle la matière de base de support de ta zone d'enrobage externe contient environ 15 % de carbonate de calcium.

10. Une composition selon la revendication 7, dans laquelle la matière de base de support de la zone d'enrobage externe contient de 2 à 6 % de Polysorbate 80® comme tensioactif non ionique et de 5 à 20 % de carbonate de calcium.

11. Une composition selon la revendication 10, dans laquelle la matière de base de support de la zone d'enrobage externe contient environ 15 % de carbonate de calcium.

12. Un procédé de fabrication d'une composition pharmaceutique sous la forme d'un comprimé à compression multiple selon l'une quelconque des revendications 1 à 11, comprenant les étapes suivantes :

(a) on prépare une Formulation (A) en mélangeant une matière de base de support et une quantité thérapeutiquement efficace comme décongestionnant d'un médicament sympathomimétique, ou d'un de ses sels pharmaceutiquement acceptables, la matière de base de support étant un mélange de (i) un ou plusieurs éthers de cellulose non ioniques solubles dans l'eau pharmaceutiquement acceptables en quantité d'environ 18 à environ 50 % en poids de la Formulation (A), (ii) un ou plusieurs tensioactifs anioniques pharmaceutiquement acceptables en quantité d'environ 2 à environ 20 % en poids de la Formulation (A) et (iii) un ou plusieurs autres excipients pharmaceutiquement acceptables,

(b) on prépare une Formulation (B) en mélangeant une seconde matière de base de support et une quantité thérapeutiquement efficace comme antihistaminique d'un pipéridinoalcanol ou d'un de ses sels pharmaceutiquement acceptables, la seconde base de support étant un mélange de (i) carbonate de calcium en quantité d'environ 0,5 à environ 25 % en poids de la Formulation (B), (ii) d'un ou plusieurs tensioactifs non ioniques pharmaceutiquement acceptables en quantité d'environ 1 à environ 10 % en poids de la Formulation (B) et (iii) un ou plusieurs autres excipients pharmaceutiquement acceptables, la Formulation (B) contenant aussi facultativement une quantité thérapeutiquement efficace comme décongestionnant d'un médicament sympathomimétique, ou d'un de ses sels pharmaceutiquement acceptables ; et

(c) on comprime plusieurs fois les Formulations (A) et (B) de manière à donner un comprimé ayant une ou plusieurs zones distinctes faites de la Formulation (A) et une ou plusieurs zones distintes faites de la Formulation (B), avec la condition que, lorsque ladite composition pharmaceutique est sous la forme d'un comprimé enrobé par compression ayant une zone de noyau interne et une zone d'enrobage externe, la zone de noyau interne est faite de la Formulation (A) et la zone d'enrobage externe est faite de la formulation (B).

**Revendications pour les Etats contractants suivants : ES, GR**

**1.** Procédé de fabrication d'une composition pharmaceutique sous la forme d'un comprimé à compression multiple comprenant

(a) une zone distincte faite d'une Formulation (A) qui comprend une matière de base de support combinée avec 1 à 200 mg d'un médicament sympathomimétique ou d'un de ses sels pharmaceutiquement acceptables, la matière de base de support étant un mélange de (i) un ou plusieurs éthers de cellulose non ioniques solubles dans l'eau pharmaceutiquement acceptables en quantité de 18 à 50 % en poids de la Formulation (A), (ii) un ou plusieurs tensioactifs anioniques pharmaceutiquement acceptables en quantité de 2 à 20 % en poids de la Formulation (A) et (iii) un ou plusieurs autres excipients pharmaceutiquement acceptables et

(b) une zone distincte faite d'une Formulation (B) qui comprend une seconde matière de base de support combinée avec 0,1 à 140 mg d'un pipéridinoalcanol ou d'un de ses sels pharmaceutiquement acceptables, la seconde base de support étant un mélange de (i) carbonate de calcium en quantité de 0,5 à 25 % en poids de la Formulation (B), (ii) un ou plusieurs tensioactifs non ioniques pharmaceutiquement acceptables en quantité de 1 à 10 % en poids de la Formulation (B) et (iii) un ou plusieurs autres excipients pharmaceutiquement acceptables, la Formulation (B) contenant aussi facultativement 1 à 200 mg d'un médicament sympathomimétique ou d'un de ses sels pharmaceutiquement acceptables,

procédé qui comprend les étapes suivantes :

(a) on prépare une Formulation (A) en mélangeant une matière de base de support et une quantité thérapeutiquement efficace comme décongestionnant d'un médicament sympathomimétique, ou d'un de ses sels pharmaceutiquement acceptables, la matière de base de support étant un mélange de (i) un ou plusieurs éthers de cellulose non ioniques solubles dans l'eau pharmaceutiquement acceptables en quantité d'environ 18 à environ 50 % en poids de la Formulation (A), (ii) un ou plusieurs tensioactifs anioniques pharmaceutiquement acceptables en quantité d'environ 2 à environ 20 % en poids de la Formulation (A) et (iii) un ou plusieurs autres excipients pharmaceutiquement acceptables,

(b) on prépare une Formulation (B) en mélangeant une seconde matière de base de support et une quantité thérapeutiquement efficace comme antihistaminique d'un pipéridinoalcanol ou d'un de ses sels pharmaceutiquement acceptables, la seconde base de support étant un mélange de (i) carbonate de calcium en quantité d'environ 0,5 à environ 25 % en poids de la Formulation (B), (ii) d'un ou plusieurs tensioactifs non ioniques pharmaceutiquement acceptables en quantité d'environ 1 à environ 10 % en poids de la Formulation (B) et (iii) un ou plusieurs autres excipients pharmaceutiquement acceptables, la Formulation (B) contenant aussi facultativement une quantité thérapeutiquement efficace comme décongestionnant d'un médicament sympathomimétique, ou d'un de ses sels pharmaceutiquement acceptables ; et

(c) on comprime plusieurs fois les Formulations (A) et (B) de manière à donner un comprimé ayant une ou plusieurs zones distinctes faites de la Formulation (A) et une ou plusieurs zones distintes faites de la Formulation (B), avec la condition que, lorsque ladite composition pharmaceutique est sous la forme d'un comprimé enrobé par compression ayant une zone de noyau interne et une zone d'enrobage externe, la zone de noyau interne est faite de la Formulation (A) et la zone d'enrobage externe est faite de la formulation (B).

2. Un procédé selon la revendication 1, dans lequel la composition est sous la forme d'un comprimé enrobé par compression.

3. Un procédé selon la revendication 2, dans lequel le médicament sympathomimétique est la pseudoéphédrine.

4. Un procédé selon la revendication 2, dans lequel le pipéridinoalcanol est l'α-[4-(1,1-diméthyléthyl)-phényl]-4-(hydroxydiphénylméthyl)-1-pipéridinebutanol.

5. Un procédé selon la revendication 2, dans lequel le médicament sympathomimétique est la pseudoéphédrine et le pipéridinoalcanol est l'α-[4-(1,1-diméthyléthyl)-phényl]-4-(hydroxydiphénylméthyl) -1-pipéridinebutanol.

6. Un procédé selon la revendication 5, dans lequel, dans la composition, la la zone de noyau interne contient environ 110 mg de chlorhydrate de pseudoéphédrine et la zone d'enrobage externe contient environ 60 mg d'α-[4-(1,1-diméthyléthyl)-phényl]-4-(hydroxydiphénylméthyl) -1-pipéridinebutanol et environ 10 mg de chlorhydrate de pseudoéphédrine.

7. Un procédé selon la revendication 6, dans lequel, dans la composition, la matière de base de support de la zone de noyau interne contient de 5 à 15 % de dodécylsulfate de sodium comme tensioactif anionique.

8. Un procédé selon la revendication 6, dans lequel, dans la composition, la matière de base de support de la zone d'enrobage externe contient de 2 à 6 % de Polysorbate 80® comme tensioactif non ionique et de 5 à 20 % de carbonate de calcium.

9. Un procédé selon la revendication 8, dans lequel, dans la composition, la matière de base de support de la zone d'enrobage externe contient environ 15 % de carbonate de calcium.

10. Un procédé selon la revendication 7, dans lequel, dans la composition, la matière de base de support de la zone d'enrobage externe contient de 2 a 6 % de Polysorbate 80® comme tensioactif non ionique et de 5 à 20 % de cabonate de calcium.

11. Un procédé selon la revendication 10, dans lequel, dans la composition, la matière de base de support de la zone d'enrobage externe contient environ 15 % de carbonate de calcium.